# EUROPEAN PATENT SPECIFICATION

(11) **EP 4 153 281 B1**
(45) Date of publication and mention of the grant of the patent: **06.11.2024**
(21) Application number: 21726234.4
(22) Date of filing: 11.05.2021
(51) Int. Cl.: A61M 5/32, A61B 17/34, A61M 25/00

(54) **ENDOLUMINAL DELIVERY CANNULA AND ASSEMBLY**
ENDOLUMINALE FREISETZUNGSKANÜLE UND ANORDNUNG
CANULE ET ENSEMBLE D'ADMINISTRATION ENDOLUMINALE

(30) Priority: 19.05.2020 SE 2050580
(43) Date of publication of application: 29.03.2023
(73) Proprietor: Smartwise Sweden AB, 146 21 Tullinge (SE)
(72) Inventor: HOLMIN, Staffan, 167 54 Bromma (SE); CLARKE, Jonathan, 112 33 Stockholm (SE); JONSSON, Stefan, 192 70 Sollentuna (SE); LUNDBERG, Johan, 187 76 Täby (SE); MUNTER, Jonny, 754 37 Uppsala (SE)
(74) Representative: Zacco Sweden AB
(86) International application number: PCT/SE2021/050443
(87) International publication number: WO 2021/235993

(56) References cited:
- EP-A1- 3 066 999
- EP-A2- 2 010 263
- WO-A1-2012/004165
- US-A1- 2005 107 751
- US-A1- 2014 094 836
- US-A1- 2016 206 832
- US-A1- 2017 100 559
- US-A1- 2018 064 914
- US-A1- 2020 113 597
- US-B1- 6 723 082

## Description

### Field of the invention

The field of the present invention relates to an endoluminal delivery device, and in particular to a endoluminal delivery device for delivery of a substance to an extravascular or intramyocardial target site, according to the preamble of the independent claim.

### Background of the invention

Within the medical field, there is a growing trend towards minimally invasive techniques when accessing or treating specific sites within the body. Such techniques have many advantages over open surgery, as they involve less trauma to the body, resulting in less complications and shorter recovery time after the procedure. Minimally invasive techniques often use the vascular system to access specific sites or regions of the body, by inserting a catheter and/or guide wire assembly percutaneously through e.g. the femoral or radial artery, and subsequently steering through the vasculature to a specific site under the guidance of angiographic imaging. However, some sites within a body are not accessible, or difficult to access, using known techniques, due to the complexity and sizing of the vasculature. Various techniques and devices are known for administration via the vascular system of substances to specific and localized target sites within the body. Examples of procedures where such techniques are used are in chemotherapy, treatment of various immunological conditions and stem cell treatments.

For example, US 8,152,758 discloses a catheter assembly including a delivery cannula with multiple channels and an expandable distal portion, wherein two needles are threaded through channels and used for infusion of a substance into the vessel wall.

US 5,464,395 shows an injection balloon catheter with side ports for needle injection into surrounding tissue.

US 2008/0319314 discloses an injection catheter with internal channels and a tip electrode, with a needle threaded through the assembly such that it can protrude out through the distal tip. Other catheter systems with injection needles are shown in e.g. US 6,613,017B1 and US 6,796,963B1

EP201 0263 A2 discloses a delivery catheter device system that is useable to deliver a substance, article or device to a location within the body of human or animal subject, wherein the delivery catheter device system generally comprises i) a catheter body having a side wall, a lumen and a distal end, ii) a tapered tip member with a pointed tip, the tip member located on the distal end of the catheter body, the pointed tip located on the distal end of the tapered tip member and iii) one or more delivery aperture(s).

These and other known systems are not adapted or suitable for access to the remote microvasculature due to their size and complexity.

Thus, the inventors of the present invention has identified a need for an improved endoluminal delivery device, which provides for enhanced access to remote locations in the body, especially via the microvasculature, as well as procedural efficiency in delivering substances to extravascular or intramyocardial target sites.

### Summary of the invention

An object of the present invention is to provide an endoluminal delivery device which allows reliable access via the vasculature to more remote extravascular sites within the body.

A further object of the present invention is to provide an endoluminal delivery device which mitigates the problems of bleeding at the puncture site of the vessel wall during and after penetration and delivery to an extravascular or intramyocardial target site.

The above-mentioned objects are achieved by the present invention according to the independent claim. Preferred embodiments are set forth in the dependent claims.

### Summary of the disclosure

According to a first aspect, an endoluminal delivery cannula, for delivery of a substance to an extravascular or intramyocardial target site via the vascular system of a human or animal is disclosed. The endoluminal delivery cannula comprises a cannula hub provided at a proximal end of the cannula and an elongated proximal portion having an outer diameter, wherein the outer diameter is being constant along essentially the entire length of the proximal portion as measured when the cannula is essentially straight. Further, the cannula comprises a tip portion arranged distally of the proximal portion and extending from the proximal portion to a distal tip of the cannula, and a continuous lumen extending from the proximal end of the cannula through the proximal portion and the tip portion to the distal tip. The tip portion has an opening at the distal tip to provide communication between the lumen and the exterior of the cannula. The tip portion is preferably tapered towards the distal tip by being provided at the proximal end of the tip portion with an outer diameter being essentially the same as the outer diameter of the elongated proximal portion, and an outer diameter at the distal tip being smaller than the outer diameter at the proximal end of the tip portion.

In some aspects, the endoluminal delivery cannula has a distal tip with a pointed tip section for penetrating tissue, wherein the pointed tip section comprises at least one primary facet and two secondary facets, wherein the two secondary facets are arranged proximally of said primary facet.

According to a further aspect, an endoluminal delivery assembly, for delivery of a substance to an extravascular or intramyocardial target site via the vascular system of a human or animal body is disclosed. The assembly comprises an endoluminal delivery cannula, a protective catheter adapted for insertion into the vascular system of a human or animal body, wherein a distal end of the assembly is configured to be guided to a position in the vascular system suitable for accessing the intended extravascular or intramyocardial target site. The assembly further comprises a proximal catheter hub provided at the proximal end of the protective catheter and adapted for guiding the catheter through the vascular system, wherein the proximal catheter hub is adapted for the endoluminal delivery cannula to be inserted therethrough and into said protective catheter.

According to yet another aspect not according to the claimed invention, a method for delivery of a substance to an extravascular or intramyocardial target site via the vascular system of a human or animal body is disclosed. The method comprises the steps of
a) providing an assembly as disclosed herein,
b) navigating a distal end of the protective catheter to a location near the extravascular or intramyocardial target site,
c) directing the distal end of the protective catheter towards a vessel wall in a general direction of the extravascular or intramyocardial target site,
d) advancing the distal tip of the endoluminal delivery cannula such that a tip of the endoluminal delivery cannula penetrates the myocardium or vessel wall and reaches the extravascular or intramyocardial target,
e) injecting the substance into the extravascular or intramyocardial target site,
f) retracting the distal tip of the endoluminal delivery cannula into the protective catheter.

### Short description of the appended drawings

Figure 1 shows an overview of use of an endoluminal delivery assembly as disclosed herein.
Figures 2A and 2B illustrates a first aspect of an endoluminal delivery cannula.
Figures 3A and 3B illustrates further aspects of an endoluminal delivery cannula.
Figures 4A and 4B illustrate a cross-sectional view of a cannula hub.
Figures 5A and 5B show perspective and side views, respectively, of an assembly comprising an endoluminal delivery cannula.
Figure 6A and 6B illustrate cross-sectional views of specific parts in an assembly comprising an endoluminal delivery cannula.
Figures 7A-7E illustrate reference planes and needle grinding angles for a cannula tip section.
Figures 8-10 show perspective views of various resulting cannula tips during experiments to determine a preferred needle grinding for a cannula tip section.
Figure 11 shows a perspective view, a top view and a side view of a preferred cannula tip section.

### Detailed description of preferred embodiments of the invention

Specific embodiments of the invention will now be described. However, it will be apparent to those skilled in the art that individual features may be combined in different manners, and the below disclosures are in no manner limiting.

The terms "proximal" and "distal" is herein used as is conventional in the art, i.e. as being in relation to a user, such that a proximal end of a device or assembly is the end directed towards the user, and a distal end is directed away from a user.

Further, the terms "cannula" and "needle" are used interchangeably herein, and both refer to an elongated tube, preferably made of a metal, which may have a sharpened tip adapted for penetration of tissue or the like.

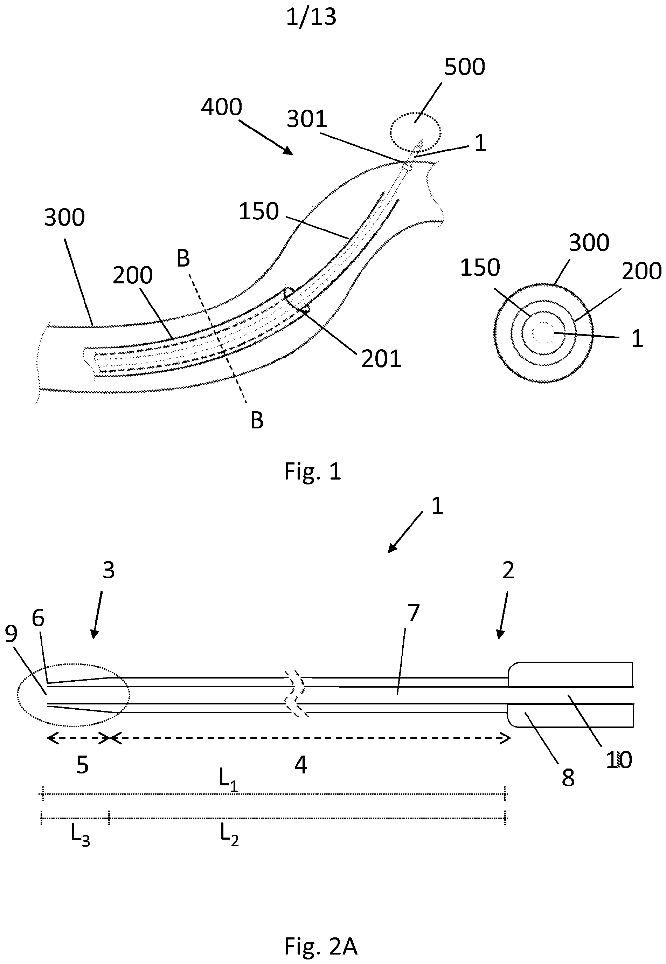

Figure 1 shows an overview of an endoluminal delivery cannula 1 as disclosed herein in use, when arranged within a blood vessel 300. The cannula 1 may preferably be provided in a kit or assembly 400 together with a protective catheter 150, and adapted for insertion into the vascular system of a human or animal body via a guide catheter, which is described in more detail further below.

To initiate a delivery procedure, a guide catheter 200 is typically used to reach a location near a defined target site 500 via the vascular system. Such a guide catheter 200 is typically a standard interventional catheter for vascular access, and may be provided together with the disclosed cannula 1 and protective catheter 150, or as a separate unit. On the right side in Figure 1, a cross-sectional view through the plane B is illustrated schematically, wherein it is seen that the protective catheter 150 is preferably coaxially arranged around the cannula 1, and the guide catheter 200 is arranged around the protective catheter 150 within the vessel 300.

The guide catheter 200 may be inserted percutaneously into a blood vessel 300 according to known techniques, e.g. using the Seldinger procedure or other known techniques, to access the vasculature via for instance the femoral artery or the radial artery. It should be noted that the devices and assemblies described herein are especially adapted for access to remote target sites in the body, i.e. for access into and via the microvasculature, and thus adapted to navigate very small vessels, as small as 1 mm or less in diameter, to reach sites within the body previously not accessible via standard techniques. However, they are also compatible for use with larger guide catheters and via larger blood vessels. The devices, assemblies and methods described herein are described in terms of reaching an extravascular target site; however, they may also similarly be used for intramyocardial delivery. Thus, a target site may be accessed via navigation through the vascular system and reached either via penetration of a blood vessel wall (for an extravascular site) or via penetration of the myocardium from e.g. inside the heart (for an intramyocardial target site).

The disclosed devices and assemblies may be used for targeted and localized delivery of one or a combination of cells, RNA, recombinant proteins, antibodies, high-dose chemotherapy, radiotherapy, or tumor-specific therapies. The specific target site 500 may be a tumor, an organ, a body cavity or a localized region of a specific tissue or body part. As further examples, disclosed devices and assemblies may be used for delivery of e.g. cell or RNA therapy for cardiometabolic regenerative therapies including the heart, liver, and kidney and for direct intra-tumor infusion.

When the guide catheter 200, which preferably has manoeuvrability and steerability properties, has been inserted via the vasculature such that the distal tip 201 of the guide catheter 200 is near the desired target site 500, the cannula 1 and protective catheter 150 are inserted via the guide catheter 200. As an alternative, the cannula 1 may be inserted into the guide catheter prior to inserting the assembly into the vasculature. In any case, the cannula 1 and protective catheter 150 are adapted to be navigated through the vascular system of a patient to a location near a target site 500 via the guide catheter 200. The cannula 1 and protective catheter 150 are directed towards the vessel wall 301, as seen in Figure 1. In some aspects, not shown in Figure 1, a guide catheter 200 with a pre-shapeable and pre-bent tip 201 may be used, such that when the tip is near the target site, the bent tip will further assist in guiding the assembly towards the vessel wall 301. Alternatively or in combination, a steerable tip 201 is also envisioned to assist in fine adjustments of the injection direction.

During the insertion through the guide catheter 200 and out towards the vessel wall 301, the protective catheter 150 and cannula 1 are prevented from axial displacement in relation to each other by a locking function at the proximal end, which will be described further below. This is to prevent the potentially sharp tip of the cannula 1 from piercing or damaging the guide catheter or the blood vessel before reaching the desired location.

Once at the desired site at the vessel wall 301, the tip of the cannula 1 is advanced out of the protective catheter 150 towards the vessel wall and further distally, such that it penetrates the vessel wall and extravascular tissue to reach the target site 500, as shown in Figure 1. In some aspects, the tip of the cannula 1 may be provided with a depth limit element, as will be described further below. The advancement of the tip may preferably also be viewed by providing one or several radiopaque markers near or at the tip, such that the tip may be located during the procedure using angiography or other imaging techniques.

Once at the target site 500, a substance to be injected into the target site is applied by syringe into the cannula via a proximal cannula hub, and ejected from the cannula via a distal opening at the tip of the cannula. In some aspects, the injected substance may be ejected both via the distal opening, and via side openings near the distal tip of the cannula. The administration of substance may be repeated a number of times, as needed. Further, the tip of the cannula may be retracted and repositioned if needed. Once administration is finished, the tip of the cannula is retracted from the vessel wall and back into the protective catheter.

As an alternative, or in addition to administrating a substance, the assembly may be used for taking samples from the target site via the tip of the cannula.

Due to a specific design of the cannula and its tip, essentially no bleeding of the vessel wall is seen. The details of the tip will described further below.

Figure 2A schematically illustrates a cross-sectional view along a longitudinal axis of the endoluminal delivery cannula 1 as disclosed herein. As described above, the cannula 1 is adapted for delivery of a substance to an extravascular or intramyocardial target site via the vascular system of a human or animal body. The cannula 1 has a proximal end 2 configured to remain outside the body and a distal end 3 configured to be inserted into the body via the vascular system to access an extravascular target site. A cannula hub 8 is preferably provided at the proximal end 2 of the cannula 1. The majority of the longitudinal length L₁ of the cannula is made up of an elongated proximal portion 4, which has a constant outer diameter D₁ along essentially the entire length of the proximal portion 4. Notably, a "constant diameter" herein means that the diameter is constant as measured when the cannula is in an elongated or essentially straight configuration, as bending may cause distortions to the diameter.

The cannula 1 further has a tip portion 5 arranged distally of the proximal portion 4 and extending from the proximal portion 4 to a distal tip 6 of the cannula 1. A continuous lumen 7 extends from an internal longitudinal channel 10 of the cannula hub at the proximal end 2 of the cannula through the proximal portion 4 and the tip portion 5 to the distal tip 6. The tip portion 5 has an opening 9 at the distal tip 6 to provide communication between the lumen 7 and the exterior of the cannula 1.

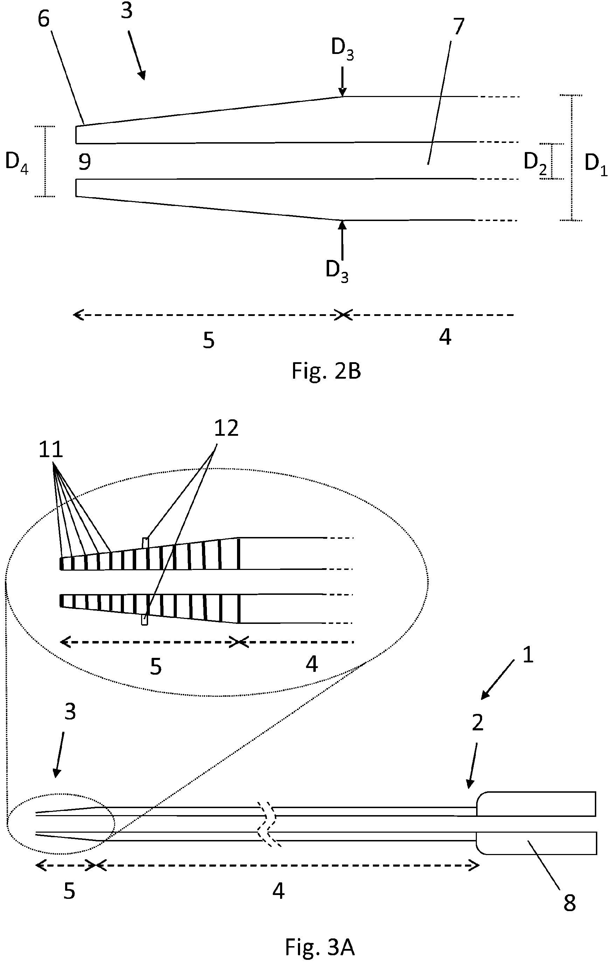

The encircled tip portion 5 in Figure 2A is shown in an enlarged and more detailed illustration in Figure 2B. The tip portion 5 is tapered towards the distal tip 6, and thus has an outer diameter D₃ at the proximal end of the tip portion 5, and another smaller outer diameter D₄ at the distal tip 6. The outer diameter D₃, is essentially the same as the outer diameter D₁ of the elongated proximal portion 4, and the outer diameter D₄ is smaller than the outer diameter D₃. In other words, a gradual taper is provided in a distal direction from the point where the proximal portion 4 transitions into the distal tip portion 5 to the distal end 6.

In some aspects, the tip portion 5 and/or proximal portion 4 may preferably be provided with one or several radiopaque marker bands 11 at predefined distances from the distal end 6. One such example is schematically shown in Figure 3A, illustrating several radiopaque marker bands 11. Such marker bands are clearly visible using angiographic imaging during a procedure, and thus used to determine the exact location of the tip and the penetration depth into a target site. Figure 3A shows only one schematic example of providing radiopaque marker bands 11. In practice, bands may be provided in any pre-defined configuration, such that they may be used for localization of the tip, and, in particular, to guide the user and determine the penetration depth from the vessel wall to the target site.

Further, in some aspects, also shown in Figure 3A, the tip portion 5 may be provided with one or several outwardly protruding depth limit elements 12, e.g. in the form of circumferential flanges or similar structures, such that a resistance is felt by the user when the depth limit elements 12 reach the vessel wall on penetration. In one aspect, a depth limit element may coincide with the transition from the tip portion 5 and the proximal portion 4. A cannula 1 may have either radiopaque markers 11 or depth limit elements 12, or both.

As is understood from the figures, and from the present disclosure, opening 9 at the distal tip 6 provides communication between the lumen 7 and the exterior of the cannula 1 and thus, when the substance to be delivered exits the cannula into the target site, the substance exits the cannula through this distal opening 9. Substance delivery is thus controlled and easily directed in the direction that the cannula is directed.

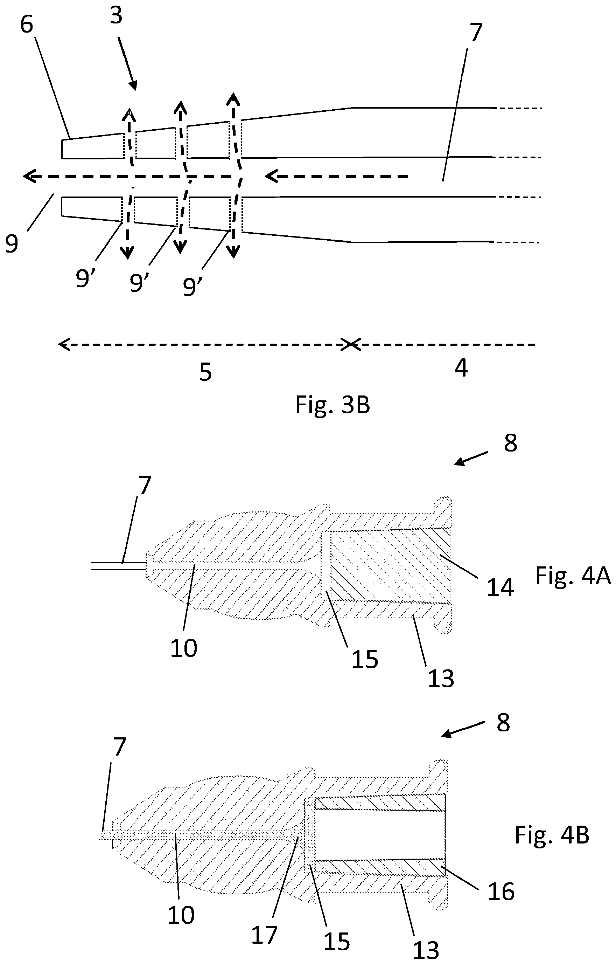

In some aspects, as illustrated in Figure 3B, the cannula may be provided with one or several secondary side openings 9', near the distal opening 9, and thus distal to any radiopaque markers 11 and/or depth limit elements 12. Such side openings allow the substance to exit the cannula through both the distal opening 9 and the one or more side openings 9', and provide a wider and/or more rapid distribution of substance at a target site, as is shown by the dashed arrows in Figure 3B. Side openings 9' allow ejection from the cannula in a radial direction, in addition to the distal direction, provided by the distal opening 9. Depending on the intended distribution, side openings 9' may be adapted to a specific pattern and/or opening size. In one aspect, one or several side openings 9' are provided along a circumference of the tip portion 5. The side openings 9' may be provided in a random pattern or a predefined pattern. In some aspects, side openings 9' are arranged near the distal opening 9. Notably, the aspects described in correlation to Figures 3A and 3B may thus be combined.

As detailed in Figure 2A, and applicable for all cannulas disclosed herein, the cannula 1 may have a total longitudinal length L₁ from the proximal end 2 to the distal end 3 within in the range of approximately 300 mm to 2500 mm. In some aspects, mainly for use in adult patients, the total longitudinal length L₁ is preferably between 1200 mm to 1900 mm, or more preferably 1650 mm to 1750 mm. However, for e.g. paediatric use a total longitudinal length L₁ of approximately 300 mm to 800 mm is preferred.

The longitudinal length L₂ of the proximal portion 4 may be within in the range of approximately 1000 mm to 2000 mm, preferably between 1200 mm to 1700, more preferably 1400 mm to 1500 mm.

The tapered tip portion 5 has a longitudinal length L₃ of at least 100 mm, preferably within in the range of 100 mm and 300 mm, more preferably between 200 mm and 280 mm.

In one aspect, the total length L₁ of the cannula 1 is approximately 1700 mm, wherein the proximal portion 4 has a longitudinal length L₂ of approximately 1450 mm and the tip portion 5 has a longitudinal length L₃ of approximately 250 mm.

In another aspect, the total length L₁ of the cannula 1 is approximately 1700 mm, wherein the proximal portion 4 has a longitudinal length L₂ of approximately 1695 mm and the tip portion 5 has a longitudinal length L₃ of approximately 5 mm. Such a cannula would thus have essentially no or minimal tapered portion.

In yet another aspect, the total length L₁ of the cannula 1 is approximately 500 mm, wherein the proximal portion 4 has a longitudinal length L₂ of approximately 425 mm and the tip portion 5 has a longitudinal length L₃ of approximately 75 mm. Such a size would be useful for paediatric use.

The proximal portion 4 of the cannula 1 preferably has a constant outer diameter D₁ within the range of 0,15 mm to 0,50 mm, preferably between 0,20 mm and 0,35 mm, more preferably between 0,25 mm to 0,28 mm.

The inner lumen 7 preferably has an inner diameter D₂ which is constant along essentially the entire length of the cannula 1, i.e. through the proximal portion 4 and the tip portion 5. Naturally, the inner diameter D₂ must be adapted to a suitable outer diameter D₁ of the cannula. The inner diameter D₂ of the lumen is preferably within in the range of approximately 0,08 mm to 0,40 mm, preferably between 0,10 mm and 0,25 mm, more preferably between 0,12 mm and 0,16 mm.

As mentioned, the outer diameter D₁ of the proximal portion 4 is preferably essentially constant along essentially the entire length of the proximal portion 4. Further, the part of the cannula where the proximal portion 4 adjoins the tip portion 5 has an outer diameter D₃ being essentially the same as the outer diameter D₁ of the elongated proximal portion 4. In other words, the cannula preferably has a smooth transition in outer diameter from the proximal portion 4 to the tip portion 5. Thereafter, the tip forms a gradual tapered tip towards the distal end 6, such that the outer diameter D₄ at the distal end 6 is smaller than the outer diameters D₃ and D₁. This taper is preferably provided such that the outer diameter D₄ at the distal tip 6 is preferably between 0,10 mm and 0,25 mm, and more preferably between 0,15 mm to 0,22 mm.

In one aspect, the outer diameter D₁ of the proximal portion 4 may be approximately 0,25 mm, the inner diameter D₂ of the lumen 7 approximately 0,134 mm and the outer diameter D₄ at the distal end 6 is 0,190 mm.

The gradual tapered tip portion 5 provides improved manoeuvrability, trackability and mainly pushability of the cannula tip, as well as providing a gradual transition to a smaller size distal tip. A smaller size tip inflicts less trauma on the vessel wall during penetration, and the small diameter of the tip allows the vessel wall to close in on itself after withdrawal of the tip, such that less bleeding is experienced after delivery. Thus, the configuration of the tip portion mitigates the need for any separate closure steps of the penetration site of the vessel wall.

The elongated proximal portion 4 and tip portion 5 of cannula 1 may preferably be made of stainless steel, nitinol, or any alloy with superelastic properties, such as Fe-Co-Ni-Ti alloys. In one aspect, the elongated proximal portion 4 and tip portion 5 are made entirely of nitinol or other nickel-titanium alloy. In another aspect, the tip portion 5 may be made of nitinol and the proximal portion 4 made of stainless steel. In further aspects, the tip portion may be made of nitinol with a tip made of a suitable ceramic material. Nitinol's superelastic properties resulting in superior flexibility provides improved navigation through small and tortuous vessels. Having a more rigid distal tip, such as a ceramic tip on a superelastic tip portion, improves the ease of penetration of the distal tip.

As seen in Figure 2A and 3, the cannula 1 is provided with a proximal cannula hub 8. Such a cannula hub 8 is adapted for delivery of a substance, from a syringe or via another adaptor or connector, to the inner lumen 7 of the cannula for further delivery to the target site at the distal end of the cannula 1. The devices and assemblies described herein are especially adapted for delivery of very small volumes of various substances. As mentioned before, substances may include chemotherapy, stem cells, RNA, orphan drugs etc., and such substances are typically very expensive. Thus, achieving a delivery system adapted for minimal loss of volume during delivery has great value. A further advantage of providing a delivery system with minimal dead volume within the system is that the risk of ejecting air bubbles into the target site is minimized, thus mitigating any air embolisms, which could cause a stroke or cardiac arrest, depending on the location within the body.

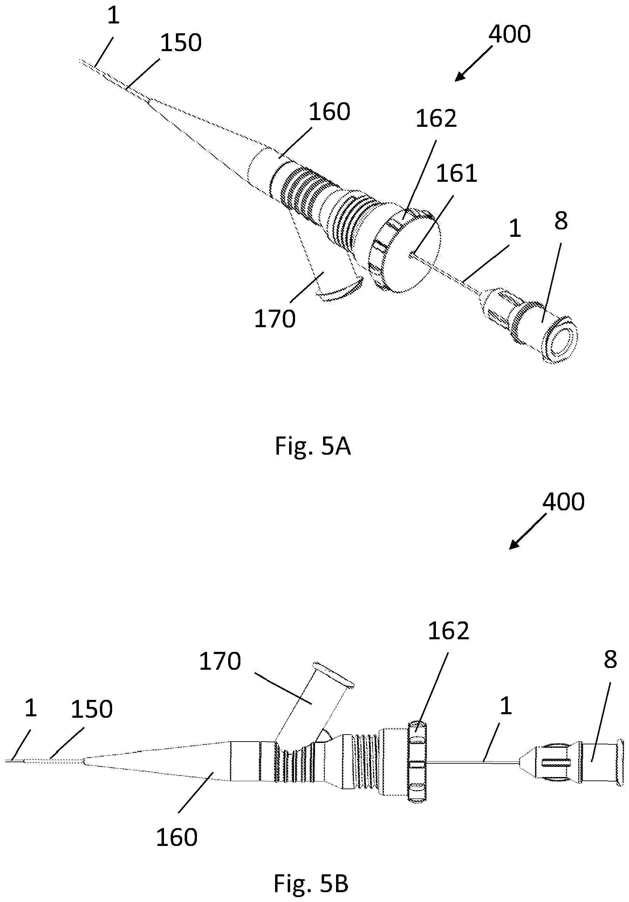

A cross-sectional view along a longitudinal axis of a preferred aspect of a cannula hub 8 is illustrated in Figure 4A. Notably, Figures 4A and 4B are cross-sectional views along a longitudinal axis of an essentially cylindrical or conical cannula hub. In other words, the cannula hub 8 preferably has a round shape being symmetric in all radial directions, as is also seen in Figures 5A and 5B, from perspective views. As seen in Figure 4A, the internal longitudinal channel 10 of the cannula hub 8 connects the lumen 7 of the proximal part 4 of the cannula to the female connector 13 at the proximal end of the cannula hub 8. The female connector 13 may be a standard Luer female connector, or other suitable connector. Before use in delivery of a substance, the cannula hub 8 may be provided with a cap or stopper 14, to keep the internal area clean and contaminant free.

The inner cavity 15 of the cannula hub 8 is preferably adapted for minimal dead volume during delivery when using a standard Luer connector 16, as shown in Figure 4B. "Dead volume" is herein used as a term for the volume of e.g. a substance which does not exit the device during delivery, e.g. the volume that remains in the cannula hub (or the system as a whole) after a syringe is pressed into the female connector 13 and a substance 17 is injected via the syringe to the cannula. In other words, the inner cavity 15 of the cannula hub 8 is configured such that when a standard male Luer connector 16 is connected to the cannula hub it fits tightly along the inner walls of the cavity 15, and a minimal inner volume is present distally of the male connector 16. The shape of the inner volume may preferably be as illustrated in Figure 4A and 4B. Thus, the inner volume of the cannula hub 8 may be formed of a thin cylindrical disc distally of the male connector 16, a cone shaped volume and a narrow cylindrical volume of the inner longitudinal channel 10. The dead volume formed by the inner volume of the cannula hub 8 is preferably less than 0,45 ml, preferably within the range of 0,25 ml to 0,45 ml, more preferably between 0,30 ml to 0,40 mm.

The dead volume of the delivery system as a whole comprises the inner volume of the cannula hub 8, as described above, together with the inner volume of the rest of the cannula 1. A small dead volume of the delivery system, as seen when a male Luer connector is attached, allows minimal loss of substance during delivery, which is particularly important when delivering expensive and/or rare substances, and minimizes the risk of creating air embolisms. Thus, preferably the total inner deadspace of the cannula hub 8 and cannula 1 is below 0,50 ml, more preferably below 0,40 ml.

A further advantage of the particular inner volume shape of the cavity of the cannula hub as shown in Figures 4A and 4b, i.e. a thin cylindrical disc transitioning smoothly into the inner longitudinal channel 10 via a short cone shape, is that less turbulence is seen in the solution when injecting the substance. This is of particular importance when using delicate substances such as solutions containing living cells, e.g. stem cells, or other less stable cells or molecules.

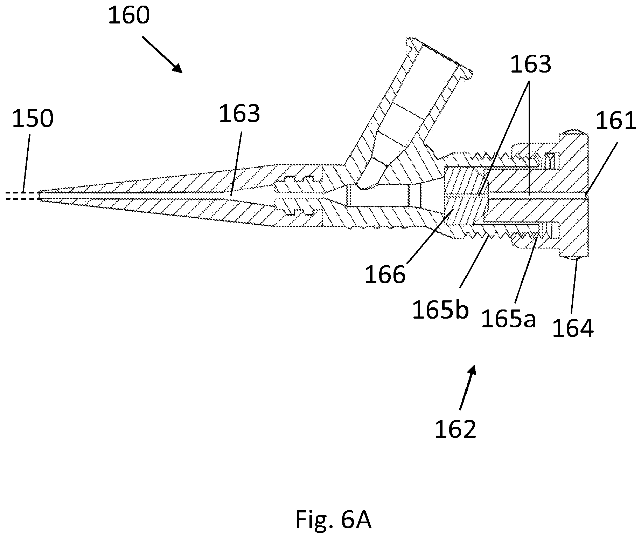

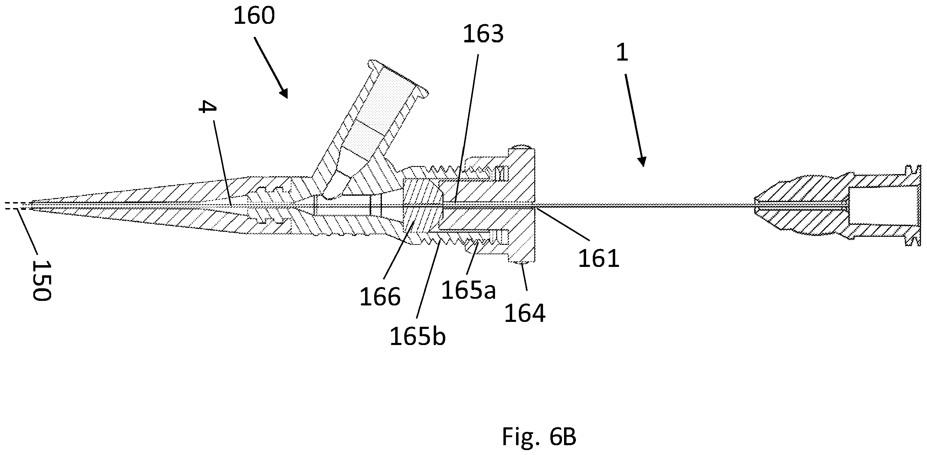

The cannula 1 and cannula hub 8 are preferably used together with a protective catheter 150 and catheter hub 160. One such assembly 400 is illustrated in Figure 5A, in perspective view, and in Figure 5B, in a side view. The protective catheter 150 is integrated with or attached to the catheter hub 160 at the distal end of the catheter hub 160. In figures 5A and 5B protective catheter 150 is shown in cut-away view; however, it extends to cover the entire cannula 1 to the distal end of the cannula. Figure 6A illustrates a cross-sectional view along the longitudinal axis of the catheter hub 160. Figure 6B illustrates the catheter hub 160 as shown in Figure 6A with a cannula 1 inserted.

The cannula 1 is adapted to be inserted from the proximal end of the catheter hub 160 through opening 161. A locking means 162 is adapted to be used to lock the cannula 1 in place after insertion of the cannula into the protective catheter 150 and during different stages of the delivery procedure. When the locking means 162 is in a locked state, all axial movement between the protective catheter 150 and the cannula 1, and thus also the cannula hub 8 and the catheter hub 160, is prevented. Before a delivery procedure, the cannula 1 is inserted into the catheter 150 via the catheter hub 160 either by the user, or during manufacture of the assembly, such that the distal tip 6 of the cannula 1 is protected by a distal end of the protective catheter 150 (not illustrated). Notably, in Figures 5A and 5B the distal end of the protective catheter is not shown, in order to illustrate that the cannula is located inside the catheter. Further, only a short proximal part of both the cannula and protective catheter 150 are illustrated in the Figures 5A and 5b.

As seen in Figures 5A and 5B, the catheter hub 160 may comprise a side port 170, which may be used for flushing the system with saline or other suitable solutions, before, during or after the delivery procedure.

As is shown in the cross-sectional view of Figure 6A, the catheter hub 160 comprises an internal channel 163 which extends from the proximal opening 161 along the longitudinal axis into the protective catheter 150 at the distal end of the catheter hub 160. As is seen in Figure 6B, the internal channel 163 is adapted for insertion of the cannula 1.

The locking means 162 may comprise any suitable mechanism to be able to lock the cannula in place when inserted into the catheter hub. Preferably, the locking means 162 is adapted to reversibly alternate between a locked state and an unlocked state. Non-limiting examples include screw locks, snap locks, friction locks and lever-based locks. Figures 5A-5B and 6A-6B show one example of a locking means 162. At the proximal end of the catheter hub 160, a locking wheel 164 comprising an extension of internal channel 163 is provided. Locking wheel 164 is provided with internal threads 165a, which are adapted to collaborate with external threads 165b on the catheter housing. When the locking wheel 164 is screwed in a distal direction, an internal locking gasket 166 is compressed and the internal channel 163 will be compressed within the gasket 166. Thus, when the cannula 1 is present in the internal channel 163, as is seen in Figure 6B, it will be gripped by the gasket 166 as the gasket 166 is compressed. Thus, the cannula 1 will be held in place by the gasket 166, thereby hindering any relative axial movement between the catheter hub 160 and cannula 1, and in so doing also any axial movement between the catheter 150 and the cannula 1. By unscrewing the locking wheel 164, the cannula 1 may be released and thereafter repositioned and optionally locked again. Thus, by using locking means 162, the relative axial position of the cannula 1 in relation to the catheter 150 may be adjusted. Further, when locked in relation to each other, the entire assembly may be steered and maneuvered as a single unit.

As described above, before employing the assembly 400 of protective catheter 150 with attached catheter hub 160, and cannula 1 with attached cannula hub 8, a guide catheter is usually placed in a vessel such that the distal end of the guide catheter is as close to the target site as possible. The assembly 400 is inserted into the guide catheter 200 and pushed or guided into a position such that the distal end of the catheter 150 protrudes from the guide catheter 200, as seen in Figure 1. During this step, the cannula 1 and protective catheter 150 are locked such that axial movement in relation to each other is prevented. This is to inhibit the potentially sharp tip of the cannula 1 from piercing or damaging the guide catheter 200 during insertion, and also to not damage the blood vessel before reaching the desired location. Preferably, the distal end of the assembly 400 is positioned such that the longitudinal axis at the tip of the cannula 1 is directed towards the target site 500, as illustrated. In some aspects, this may be achieved by using a guide catheter 200 with a preshaped bent tip and/or a steerable tip 201, as is known in the art.

Once the distal tip 201 is directed towards the vessel wall and the target site 500, the assembly 400 is advanced distally out of the guide catheter 200. At this stage, the distal tip 6 of the cannula 1 is still contained within the protective catheter 150, to avoid any unintended damage to the vessel.

Thereafter locking means 162 of the catheter hub 160 is released, and the tip of the cannula 1 is advanced out of the protective catheter 150 towards the vessel wall and further distally, such that it penetrates the vessel wall and extravascular tissue to reach the target site. This movement is performed by moving the proximal cannula hub 8 closer to the proximal end of the catheter hub 160, by e.g. holding the catheter hub 160 still and moving the cannula hub 8 distally.

In some aspects, a stop element may be provided to prevent premature advancement of the distal tip before reaching a desired location. Such a stop element could be a stop ring or similar arrangement around the cannula 1 between the cannula hub 8 and the catheter hub 160, that may be manually removed before penetration of the vessel wall. As an alternative, or in combination with a stop element, a marker may be provided on the cannula 1 at a location such that it can be seen between the cannula hub 8 and locking means 162 of the catheter hub 160 when the distal tip 6 of the cannula is protected by the distal tip of the protective catheter 150. Such a marker provides a user with a visual indication of when the sharp tip is in a retracted and protected position within the protective catheter, and is useful both during initial positioning of the tip and when repositioning the endoluminal delivery device.

At any desired time during delivery of a substance, such as when the cannula tip protrudes from the catheter tip, the cannula 1 and catheter 150 may be locked in a relative axial arrangement, e.g. by engaging locking means 162. After delivery, or if the cannula tip is to be repositioned, the procedure may be reversed such that the cannula tip is once more protected by the catheter tip, and thereafter optionally repeated for another delivery dose. As described above, in some aspects the distal tip portion 5 is preferably gradually tapered towards the distal tip 6.

The distal tip portion 5 of the cannula is provided with a pointed tip section 100 for penetrating tissue formed by at least one primary facet F₁ and two secondary facets F₂ and F₃. Notably, herein such a tip section is shown on a cannula 1 for delivery of a substance via the vasculature. However, it is also conceivable to use a similar pointed tip for other devices with similar use, such as micro-needles for intramuscular or intradermal injections.

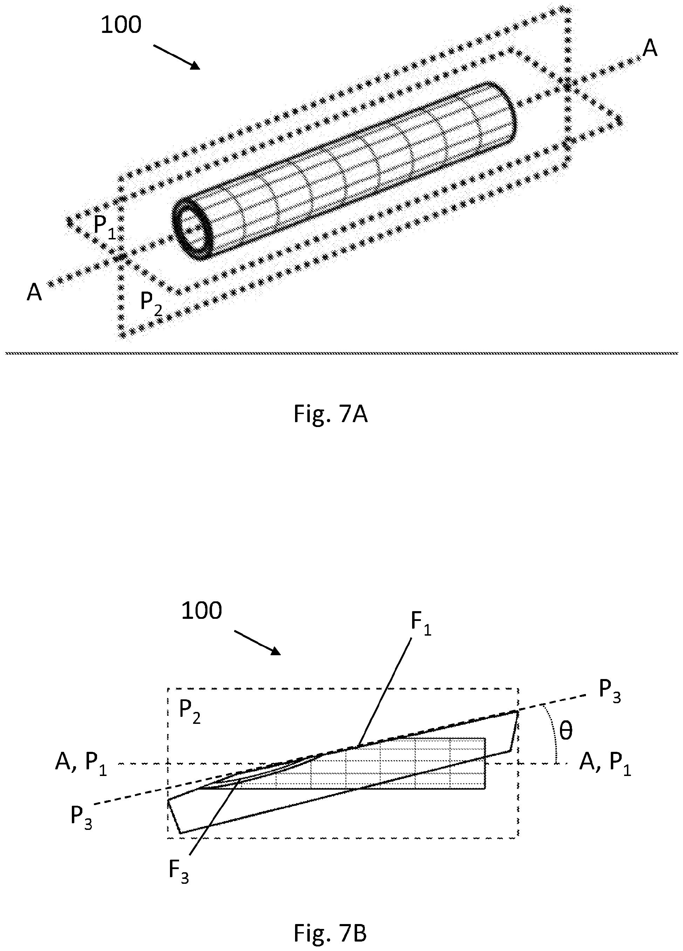

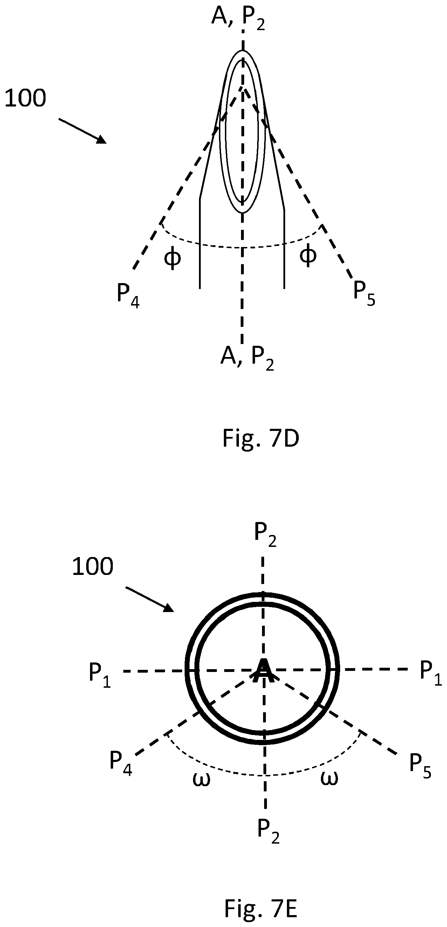

Figures 7A-7E illustrate the grinding angles used to form the primary and secondary facets. Notably, the gradual taper of the distal tip portion 5 of the cannula is not visible in Figures 7A-7E, and the following figures, as they schematically illustrate only the final approximate 1-3 millimetres of the tip section 100. Furthermore, in figures 7A, 7B, 7C and 8 to 11, i.e. when showing a perspective or side view, the left side of the figure is directed generally in the distal direction, and the right side of the figure is directed generally towards the proximal end of the cannula. In Figure 7D, showing a top view of a tip section 100, upwards in the figure corresponds to a distal direction of the tip. In Figure 7E, a tip section 100 is viewed along the longitudinal axis A.

In the context of needle grinding, i.e. forming a sharpened tip from a hollow cylindrical cannula, the distal end of the cannula is ground down and sharpened against a grinding wheel or other grinding media. Normally, the grinding wheel is stationary, and the needle or cannula is applied at a fixed angle in relation to the grinding surface. The resulting facets or bevels are thus formed in one or several planes which may be defined in relation to the geometry of the cannula itself.

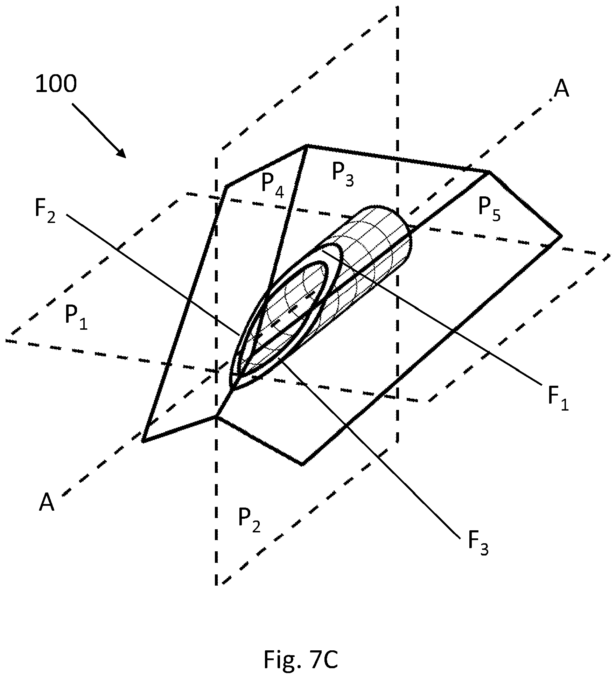

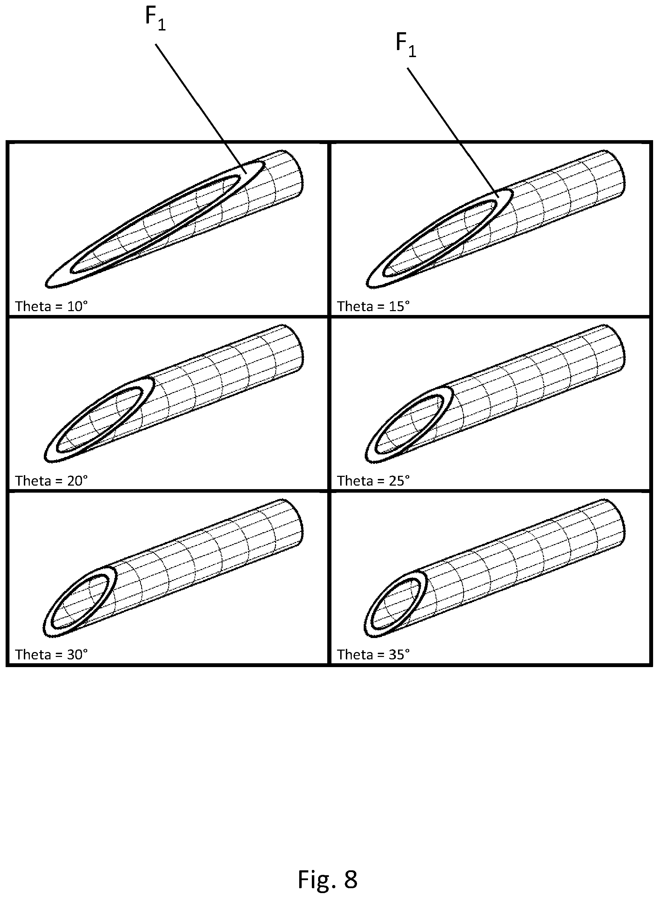

Figure 7A shows a perspective and schematic view of a tip section 100 before grinding of any facets. The cylindrical cannula tip section 100 is illustrated to have two reference planes, shown as a first plane P₁ and a second plane P₂ arranged along the longitudinal axis A, wherein the first and second planes P₁, P₂ are perpendicular to each other. These reference planes P₁, P₂ are herein used to define the angles and placement of the planes defining the facets of the resulting tip section 100. For ease of understanding, the first plane P₁ may be viewed as a horizontal plane and the second plane P₂ may be viewed as a vertical plane. Figure 7B illustrates a side view of the tip section 100 shown in Figure 7A after grinding of the facets. A perspective view of the tip section 100 is shown in Figure 7C, wherein the planes forming the facets in relation to the reference planes are illustrated, as will be detailed below. In a first aspect, the primary facet F₁ of the tip is formed by a needle grinding in a third plane P₃, wherein the third plane P₃ is positioned at an angle theta θ, in respect to the first plane P₁, as best seen in Figure 7B. Preferably, the third plane P₃ is symmetrically arranged in relation to the second plane P₂ such that the third plane P₃ intersects the first plane P₁, in an extension perpendicular to the second plane P₂. In other words, a pointed tip 6 is formed in the distal direction. Examples of a resulting tip after the first grinding are shown in Figure 8 and discussed further below.

Following the formation of the primary facet F₁, two secondary facets, F₂ and F₃, are formed by a needle grinding of the distal tip in a fourth and fifth plane, P₄ and P₅, respectively, as is illustrated in Figure 7C. The arrangement of fourth and fifth planes P₄, P₅ is further explained by way of Figure 7D and 7E.

Figure 7D illustrates a top view, i.e. as seen from a direction perpendicular to plane P₁, of the tip section 100 after a first grinding in the third plane P₃. Figure 7E shows the tip section 100 from a distal direction, along the longitudinal axis A.

The fourth and fifth planes P₄ ,P₅ are arranged at a set of two symmetrical and combined angles, such that the fourth and fifth planes P₄ ,P₅ are symmetrically arranged in relation to the longitudinal axis, and also to the first and second planes P₁, P₂. The symmetrical angles of the fourth and fifth planes P₄ ,P₅ are thus comprised of two combined angles measured in different planes or views. As seen in Figure 7D, when the tip section 100 is viewed from a top view, or along plane P₂, a first angle phi (p may be measured on either side of the second plane P₂ in the first plane P₁.

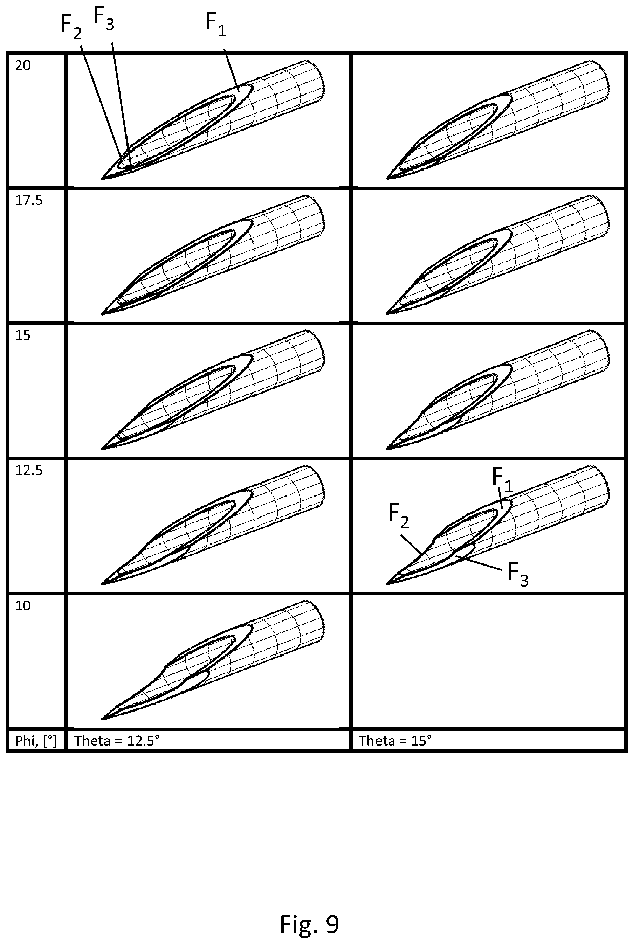

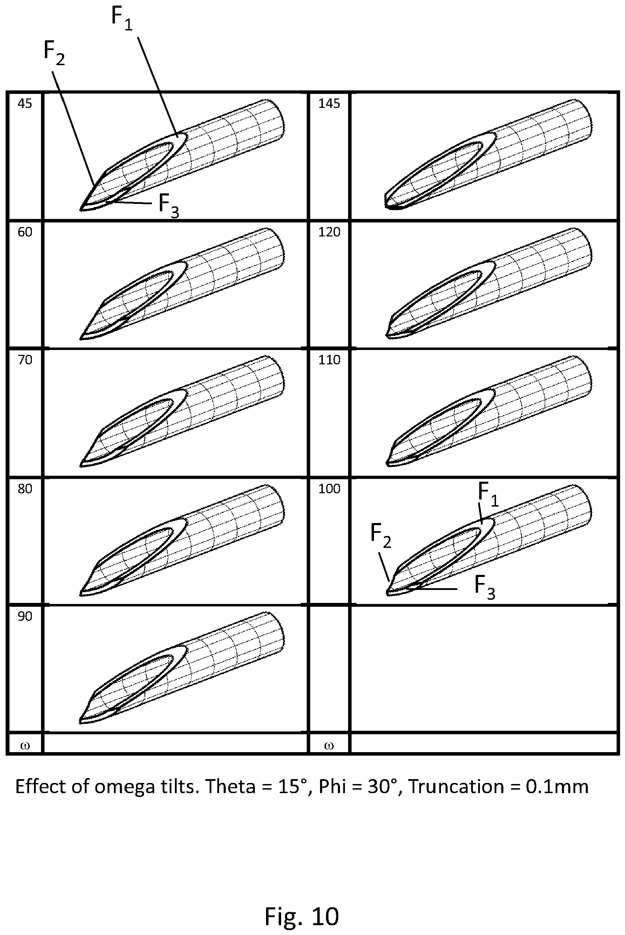

The second component of the arrangement of the fourth and fifth planes P₄ ,P₅ is a rotational angle omega ω around longitudinal axis A, as illustrated in Figure 7E. Figure 7E illustrates a tip section 100 as seen along the longitudinal axis A. The fourth and fifth planes P₄ ,P₅ are defined by rotation in two opposite directions in relation to the reference planes. Thus, the two angles phi ϕ and omega ω together describe the arrangement of fourth and fifth planes P₄ ,P₅, and thus the angles defining the two secondary facets F₂ and F₃. Examples of several resulting tips after the different grinding steps are shown in Figures 9 and 10, and are further described in the experimental section below.

As is evident from the above and seen in the figures, the two secondary facets F₂, F₃ form the distal tip 6 together with an outer mantle surface of the tip section 100. Hence, the sharpness of the distal tip may be controlled by both phi ϕ and omega ω, thereby offering the ability to optimize sharpness and finding the most effective penetration of e.g. a tissue.

It has been found by the inventors that if phi ϕ is larger than theta θ, a more suitable geometry is obtained. However, if phi ϕ is larger than 45 degrees, the tip becomes too blunt.

In addition, especially when providing the one primary facet F₁ at angle theta θ and two secondary facets F₂ and F₃, at angles phi ϕ and omega ω as described above, it is apparent that theta must be low, preferably under 30 degrees, to obtain a usable tip at all. However, as is described in the experiments below, if theta θ is under 10 degrees, then the tip will have an unsatisfactory rigidity.

Thus, through extensive testing, calculation and inspection of resulting tips the present inventors have reached the conclusion that in order to obtain an improved tip for penetration of a blood vessel wall and surrounding tissue with minimal trauma, leading to minimal bleeding on removal, as well as a needle tip suitable for multiple penetration procedures, the following criteria are preferable. A needle tip section with one primary facet F₁ and two secondary facets F₂ and F₃ is preferably provided at primary facet angle theta θ being between 10.0 and 20.0 degrees, and secondary facets provided at +/- phi ϕ angles between 15.0 and 20.0 degrees, and +/- omega ω angles between 25.0 and 90.0 degrees.

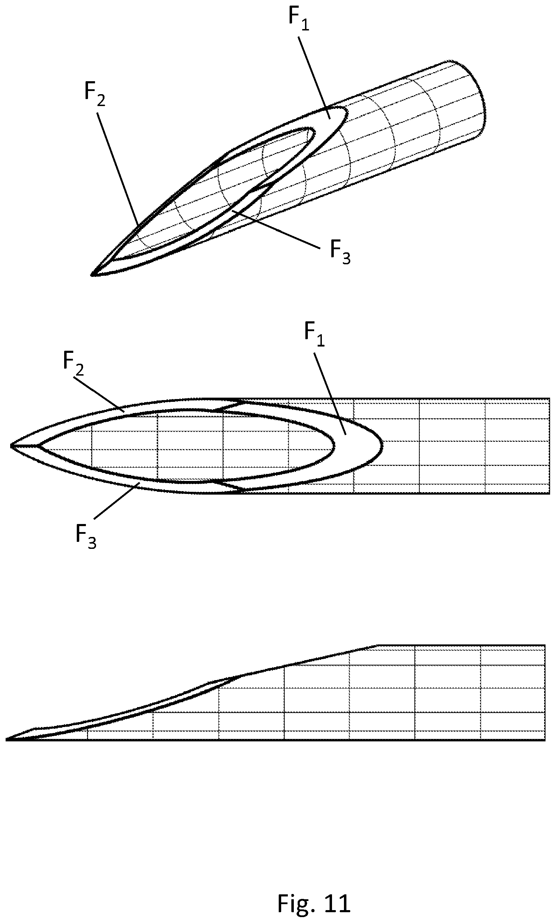

As one example, a needle tip section is shown in Figure 11, having theta θ angle 12.5 degrees, phi ϕ angle +/-18.0 degrees, omega angles +/-30.0 degrees.

Another example of a suitable tip section would be a tip having theta θ angle 15.0 degrees, phi ϕ angle +/-20.0 degrees, omega angles +/-30.0 degrees.

It has thus been found by the inventors, that the combined effect of controlling the intersection between F₂ and F₃ by both phi ϕ and omega ω as described herein results in a triangular point with optimum sharpness and effective penetration of e.g. tissue, with minimal bleeding.

The above described dimensions and configuration of a tip section 100 works in combination with the overall tapered tip portion 5 of the cannula 1, such that a less-traumatic penetration of a vessel wall may be achieved, mitigating the need to provide any specific closure measures, such as plugging or stopping the hole made by the penetrating tip in the vessel wall.

### Experiments

Experiments were performed to determine the preferred shape of needle tip for optimal rigidity.

The figures illustrating a needle tip (Figures 8 to 11) are based on a catheter having ID (inner diameter) 0.147 mm and OD (outer diameter) 0.190 mm at the distal end, being tapered by a 25 cm long grinding. Based on the dimensions of the catheter, the optimal grinding angles were mathematically modelled to determine low penetration force and low buckling of the needle tip, i.e. rigidity.

### First grinding angle, theta

The first grinding angle, theta, was evaluated for angles between 10° and 35°. These grinding angles, as illustrated in Figure 8, yielded a soft, rounded point, which had low vertical rigidity, but good lateral rigidity.

### Second- and third grindings, phi

To improve the tip sharpness and to improve vertical rigidity, two symmetric grindings at angle phi to the catheter axis were evaluated, as illustrated on Figure 9. However, during these grindings, the tip inevitably becomes truncated. In addition, at certain angles truncation produces a tip with a vertical edge instead of a triangular point when the truncation is >0.3mm. This would reduce the sharpness.

### Combined effect of theta and phi for 0.1mm truncation

By setting a fixed truncation of 0.1mm to maintain minimal truncation, the effects of theta and phi were explored, as shown in Figure 10. From such models, it can be seen that more truncation is allowed for lower values on theta and phi should be larger than theta for a suitable geometry. In addition, theta must be low in order to produce a usable tip at all. Values of 30° or 45° are not recommendable.

### Combined effect of theta and phi without truncation

With the option of high precision grinding, truncation can be limited. As a result, the effect of theta and phi was evaluated for an ideal situation for more suitable ranges of theta and phi. In this modelling, the tips are all sharp as all the needle facets meet in a point. When phi<=theta, the ID (inner diameter) surface is cut by facets 2 and 3 and produces a thin tongue, as seen in Figure 9. However, when phi is a bit larger than theta, facets 2 and 3 do not intersect the inner surface which improves the tip rigidity, i.e. the flexure strength in the up/down direction.

### Effect of omega tilts

Facets 2 and 3 can be tilted by rotating the catheter by an angle, omega, as shown in Fig. 7D. Consequently, the catheter was rotated +/- omega when grinding facets 2 and 3 respectively.

The corners between facets 1&2 and 2&3 cannot be removed by the tilted grinding with a truncation of 0.1mm when theta and phi are set at 20° and 30° respectively as shown in Figure 10.

### Region of good design

To determine a beneficial geometry to limit corners where the facets meet, omega was set to 30°, and a series of different geometries was constructed and inspected, after which a domain of good design could be identified. Theta < 10° produced a needle tip with low rigidity. When phi > 45°, the tip will not become sharp.

### Analyses of rigidity

The modelled angles were then compared to the ground needle tip, and the grinding angles of the ground needle tip were very close to the intended values calculated by the modelling. By setting an elastic constant of E= 80GPa and a value of 0.3 to Poisson's ratio (common for most metals), the force necessary to deflect the tip was calculated. It was discovered that the truncation by grinding 2 and 3 (phi angles) had a major impact on the tip rigidity.

### Finding the optimum design

A series of calculations was performed where theta ranged between 10° and 20°, phi ranged between 15° and 20°, omega was set at 30°, and truncation was 0.0 to 0.1 mm to construct contour lines of buckling forces needed to deflect the outermost tip upwards a complete outer diameter of the cannula.

A limit of good design was constructed to find the angles for optimal rigidity and found to be theta = 12.5°, phi = 18°, omega =30° and a truncation of 0.1mm. The suggested optimum design is shown in Figure 11, as detailed above.

The present invention is not limited to the above-described preferred embodiments. Therefore, the above embodiments should not be taken as limiting the scope of the invention, which is defined by the appending claims.

## Claims

1. An endoluminal delivery cannula (1), for delivery of a substance to an extravascular or intramyocardial target site via the vascular system of a human or animal body,
said cannula (1) having a proximal end (2) configured to remain outside the body and a distal end (3) configured to be inserted into the body via the vascular system to access the extravascular or intramyocardial target site, said cannula (1) having a total longitudinal length (L₁) from said proximal end (2) to said distal end (3), and said cannula (1) comprising
a cannula hub (8) provided at the proximal end (2) of the cannula (1),
an elongated proximal portion (4) having a longitudinal length (L₂) and an outer diameter (D₁), said outer diameter (D₁) being constant along essentially the entire length of the proximal portion (4) as measured when the cannula is essentially straight, and
a tip portion (5) arranged distally of the proximal portion (4) and extending from the proximal portion (4) to a distal tip (6) of the cannula (1), and
a continuous lumen (7) extending from the proximal end (2) of the cannula through the proximal portion (4) and the tip portion (5) to the distal tip (6) and said lumen (7) having an inner diameter (D₂) along the entire length of the cannula (1),
said tip portion (5) having a primary opening (9) at the distal tip (6) to provide communication between the lumen (7) and the exterior of the cannula (1),
said tip portion (5) being tapered towards the distal tip (6) along the entire tip portion (5) from the distal end of the elongated proximal portion (4) to the distal tip (6),
said tapered tip portion (5) being provided at the proximal end of the tip portion (5) with an outer diameter (D₃) being essentially the same as the outer diameter (D₁) of the elongated proximal portion (4), and an outer diameter (D₄) at the distal tip (6) being smaller than the outer diameter (D₃) at the proximal end of the tapered tip portion (5),
wherein said tapered tip portion (5) is provided with a distal pointed tip section (100) for penetrating tissue,
**characterized in that**
said tapered tip portion (5) has a longitudinal length (L₃) of at least 100 mm, preferably between 100 mm and 300 mm, more preferably between 200 mm and 280 mm,
wherein said pointed tip section (100) comprises at least one primary facet (F₁) and two secondary facets (F₂, F₃), wherein said two secondary facets (F₂, F₃) are arranged proximally of said primary facet (F₁), and
wherein said distal pointed tip section (100) also has a lumen (7) being a continuation of the continuous lumen (7).

2. The endoluminal delivery cannula (1) according to claim 1, wherein said total longitudinal length (L₁) from said proximal end (2) to said distal end (3) is within in the range of approximately 300 mm to 2500 mm.

3. The endoluminal delivery cannula (1) according to any preceding claim, wherein said outer diameter D₄ at the distal tip (6) is within the range of 0,10 mm to 0,25 mm, and preferably between 0,15 mm to 0,22 mm.

4. The endoluminal delivery cannula (1) according to any preceding claim, wherein said tapered tip portion (5) and/or proximal portion (4) is provided with one or several radiopaque marker bands (11) at predefined distances from the distal tip (6).

5. The endoluminal delivery cannula (1) according to any preceding claim, wherein said tapered tip portion (5) is provided with one or several protruding depth limit elements (12).

6. The endoluminal delivery cannula (1) according to any preceding claim, wherein said tapered tip portion (5) is further provided with one or several side openings (9') along at least part of the tapered tip portion (5).

7. The endoluminal delivery cannula (1) according to any preceding claim, wherein the cannula hub (8) is provided with an internal longitudinal channel (10) and a female connector (13) at its proximal end, said internal longitudinal channel (10) configured to provide communication between the continuous lumen (7) of the cannula and the female connector (10), wherein the internal longitudinal channel (10) and the female connector (13) together have a total inner volume being less than 0,45 ml when a corresponding male connector is attached to the female connector (13).

8. The endoluminal delivery cannula (1) according to any preceding claim, wherein
said distal tip section (100) has
a first plane (P₁) being arranged along a central longitudinal axis (A), and a second plane (P₂) being arranged along the longitudinal axis (A), said first and second planes (P₁, P₂) being perpendicular to each other, and
a third plane (P₃) being positioned at an angle theta (θ), in respect to the first plane (P₁), said third plane (P₃) being symmetrically arranged in relation to the second plane (P₂), and
a fourth plane (P₄) and fifth plane (P₅) being arranged at a set of two symmetrical angles, said angles being symmetrical in relation to each of said first and second planes (P₁, P₂),
said symmetrical angles being combined angles comprising a first angle phi (ϕ) as measured from the second plane (P₂) in the first plane (P₁), and a second angle omega (ω) being a rotational angle around longitudinal axis (A),
wherein
said primary facet (F₁) of said distal tip section (100) is provided in said third plane (P₃), and
said two secondary facets (F₂ ,F₃) are provided in said fourth and fifth plane (P₄ ,P₅), respectively.

9. The endoluminal delivery cannula (1) according to claim 8, wherein said two secondary facets (F₂, F₃) form a distal tip (6) together with an outer mantle surface of the tip section (100).

10. The endoluminal delivery cannula (1) according to any of claims 8 or 9, wherein the angle phi (ϕ) is larger than said angle theta (θ).

11. The endoluminal delivery cannula (1) according to any of claims 8 to 10, wherein said one primary facet (F₁) and two secondary facets (F₂, F₃) are provided at primary facet angle theta (θ) being between 10.0 and 20.0 degrees, and secondary facets provided at +/- phi (ϕ) angles between 15.0 and 20.0 degrees, and +/- omega (ω) angles between 25.0 and 90.0 degrees.

12. An endoluminal delivery assembly (400), for delivery of a substance to an extravascular or intramyocardial target site via the vascular system of a human or animal body, comprising
the endoluminal delivery cannula (1) according to any of claims 1 to 11,
a protective catheter (150) adapted for insertion into the vascular system of a human or animal body,
wherein a distal end of said assembly is configured to be guided to a position in the vascular system suitable for accessing the intended extravascular or intramyocardial target site, and
a proximal catheter hub (160) provided at the proximal end of the protective catheter (150) and adapted for guiding the protective catheter (150) through the vascular system, said proximal catheter hub (160) being adapted for the endoluminal delivery cannula (1) to be inserted therethrough and into said protective catheter (150).

13. The endoluminal delivery assembly (400) according to claim 12, wherein the catheter hub (160) further comprises a locking means (162) adapted to reversibly alternate between a locked state and an unlocked state, said locking means (162) configured, in a locked state, to prevent axial movement between the protective catheter (150) and the endoluminal delivery cannula (1).

14. The endoluminal delivery assembly (400) according to claim 13, wherein the locking means (162) comprises a locking wheel (164) provided with internal threads (165a) collaborating with external threads (165b) of the catheter hub housing, said the locking means adapted such that when said locking means is actuated, an internal locking gasket (166) is compressed to grip a proximal end of said endoluminal delivery cannula.

## Patentansprüche

1. Endoluminale Freisetzungskanüle (1) zum Freisetzen einer Substanz an eine extravaskuläre oder intramyokardiale Zielstelle über das Gefäßsystem eines menschlichen oder tierischen Körpers,
wobei die Kanüle (1) ein proximales Ende (2), das dazu konfiguriert ist, außerhalb des Körpers zu bleiben, und ein distales Ende (3), das dazu konfiguriert ist, über das Gefäßsystem in den Körper eingeführt zu werden, um auf die extravaskuläre oder intramyokardiale Zielstelle zuzugreifen, aufweist, wobei die Kanüle (1) eine gesamte Längslänge (L₁) von dem proximalen Ende (2) zu dem distalen Ende (3) aufweist und die Kanüle (1) Folgendes umfasst:
einen Kanülenansatz (8), der an dem proximalen Ende (2) der Kanüle (1) bereitgestellt ist,
einen länglichen proximalen Abschnitt (4), der eine Längslänge (L₂) und einen Außendurchmesser (D₁) aufweist, wobei der Außendurchmesser (D₁) entlang im Wesentlichen der vollständigen Länge des proximalen Abschnitts (4) konstant ist, wie gemessen, wenn die Kanüle im Wesentlichen gerade ist, und
einen Spitzenabschnitt (5), der distal des proximalen Abschnitts (4) angeordnet ist und sich von dem proximalen Abschnitt (4) zu einer distalen Spitze (6) der Kanüle (1) erstreckt, und
ein kontinuierliches Lumen (7), das sich von dem proximalen Ende (2) der Kanüle durch den proximalen Abschnitt (4) und den Spitzenabschnitt (5) zu der distalen Spitze (6) erstreckt, und wobei das Lumen (7) einen Innendurchmesser (D₂) entlang der vollständigen Länge der Kanüle (1) aufweist,
wobei der Spitzenabschnitt (5) eine primäre Öffnung (9) an der distalen Spitze (6) aufweist, um Kommunikation zwischen dem Lumen (7) und dem Äußeren der Kanüle (1) bereitzustellen,
wobei der Spitzenabschnitt (5) in Richtung der distalen Spitze (6) entlang des vollständigen Spitzenabschnitts (5) von dem distalen Ende des länglichen proximalen Abschnitts (4) zu der distalen Spitze (6) verjüngt ist,
wobei der verjüngte Spitzenabschnitt (5) an dem proximalen Ende des Spitzenabschnitts (5) mit einem äußeren Durchmesser (D₃), der im Wesentlichen der gleiche wie der äußere Durchmesser (D₁) des länglichen proximalen Abschnitts (4) ist, und einem äußeren Durchmesser (D₄) an der distalen Spitze, der kleiner als der äußere Durchmesser (D₃) an dem proximalen Ende des verjüngten Spitzenabschnitts (5) ist, bereitgestellt ist,
wobei der verjüngte Spitzenabschnitt (5) mit einem distalen scharfen Spitzenteil (100) zum Durchdringen von Gewebe bereitgestellt ist,
**dadurch gekennzeichnet, dass**
der verjüngte Spitzenabschnitt (5) eine Längslänge (L₃) von mindestens 100 mm, bevorzugt zwischen 100 mm und 300 mm, bevorzugter zwischen 200 mm und 280 mm aufweist,
wobei der scharfe Spitzenteil (100) mindestens eine primäre Facette (F₁) und zwei sekundäre Facetten (F₂, F₃) umfasst,
wobei die zwei sekundären Facetten (F₂, F₃) proximal der primären Facette (F₁) angeordnet sind und
wobei der distale scharfe Spitzenteil (100) ebenfalls ein Lumen (7) aufweist, das eine Fortsetzung des kontinuierlichen Lumens (7) ist.

2. Endoluminale Freisetzungskanüle (1) nach Anspruch 1, wobei die gesamte Längslänge (L₁) von dem proximalen Ende (2) zu dem distalen Ende (3) innerhalb des Bereichs von ungefähr 300 mm bis 2500 mm liegt.

3. Endoluminale Freisetzungskanüle (1) nach einem der vorhergehenden Ansprüche, wobei der äußere Durchmesser D₄ an der distalen Spitze (6) innerhalb des Bereichs von 0,10 mm bis 0,25 mm und bevorzugt zwischen 0,15 mm bis 0,22 mm liegt.

4. Endoluminale Freisetzungskanüle (1) nach einem der vorhergehenden Ansprüche, wobei der verjüngte Spitzenabschnitt (5) und/oder der proximale Abschnitt (4) mit einem oder mehreren strahlenundurchlässigen Markierungsstreifen (11) auf vordefinierten Abständen von der distalen Spitze (6) bereitgestellt ist.

5. Endoluminale Freisetzungskanüle (1) nach einem der vorhergehenden Ansprüche, wobei der verjüngte Spitzenabschnitt (5) mit einem oder mehreren hervorstehenden Tiefenbegrenzungselementen (12) bereitgestellt ist.

6. Endoluminale Freisetzungskanüle (1) nach einem der vorhergehenden Ansprüche, wobei der verjüngte Spitzenabschnitt (5) ferner mit einer oder mehreren Seitenöffnungen (9') entlang mindestens eines Teils des verjüngten Spitzenabschnitts (5) bereitgestellt ist.

7. Endoluminale Freisetzungskanüle (1) nach einem der vorhergehenden Ansprüche, wobei der Kanülenansatz (8) an seinem proximalen Ende mit einem inneren Längskanal (10) und einem Buchsenanschluss (13) bereitgestellt ist, wobei der innere Längskanal (10) dazu konfiguriert ist, Kommunikation zwischen dem kontinuierlichen Lumen (7) der Kanüle und dem Buchsenanschluss (10) bereitzustellen, wobei der innere Längskanal (10) und der Buchsenanschluss (13) zusammen ein gesamtes inneres Volumen aufweisen, das weniger als 0,45 ml beträgt, wenn ein entsprechender Steckanschluss an dem Buchsenanschluss (13) befestigt ist.

8. Endoluminale Freisetzungskanüle (1) nach einem der vorhergehenden Ansprüche, wobei der distale Spitzenteil (100) Folgendes aufweist:
eine erste Ebene (P₁), die entlang einer zentralen Längsachse (A) angeordnet ist, und eine zweite Ebene (P₂), die entlang der Längsachse (A) angeordnet ist, wobei die erste und die zweite Ebene (P₁, P₂) senkrecht zueinander sind, und
eine dritte Ebene (P₃), die in einem Winkel Theta (θ) in Bezug auf die erste Ebene (P₁) positioniert ist, wobei die dritte Ebene (P₃) symmetrisch in Bezug auf die zweite Ebene (P₂) angeordnet ist, und
eine vierte Ebene (P₄) und eine fünfte Ebene (P₅), die als ein Satz zweier symmetrischer Winkel angeordnet sind, wobei die Winkel in Bezug auf jede der ersten und der zweiten Ebene (P₁, P₂) symmetrisch sind,
wobei die symmetrischen Winkel kombinierte Winkel sind, die einen ersten Winkel Phi (ϕ), wie gemessen von der zweiten Ebene (P₂), in der ersten Ebene (P₁) und einen zweiten Winkel Omega (ω) umfassen, der ein Drehwinkel um die Längsachse (A) ist, wobei
die primäre Facette (F₁) des distalen Spitzenteils (100) in der dritten Ebene (P₃) bereitgestellt ist und
die zwei sekundären Facetten (F₂, F₃) in der vierten beziehungsweise der fünften Ebene (P₄, P₅) bereitgestellt sind.

9. Endoluminale Freisetzungskanüle (1) nach Anspruch 8, wobei die zwei sekundären Facetten (F₂, F₃) zusammen mit einer äußeren Hüllenfläche des Spitzenteils (100) eine distale Spitze (6) bilden.

10. Endoluminale Freisetzungskanüle (1) nach einem der Ansprüche 8 oder 9, wobei der Winkel Phi (ϕ) größer als der Winkel Theta (θ) ist.

11. Endoluminale Freisetzungskanüle (1) nach einem der Ansprüche 8 bis 10, wobei die eine primäre Facette (F₁) und die zwei sekundären Facetten (F₂, F₃) in einem primären Facettenwinkel Theta (θ) bereitgestellt werden, der zwischen 10,0 und 20,0 Grad beträgt, und die sekundären Facetten bei +/-Phi-Winkeln, ϕ-Winkeln, zwischen 15,0 und 20,0 Grad und +/-Omega-Winkeln, ω-Winkeln, zwischen 25,0 und 90,0 Grad bereitgestellt werden.

12. Endoluminale Freisetzungsanordnung (400) zum Freisetzen einer Substanz an eine extravaskuläre oder intramyokardiale Zielstelle über das Gefäßsystem eines menschlichen oder tierischen Körpers, umfassend:
die endoluminale Freisetzungskanüle (1) nach einem der Ansprüche 1 bis 11,
einen Schutzkatheter (150) der zum Einführen in das Gefäßsystem eines menschlichen oder tierischen Körpers ausgelegt ist,
wobei ein distales Ende der Anordnung dazu konfiguriert ist, an eine Position in dem Gefäßsystem geleitet zu werden, die zum Zugreifen auf die angestrebte extravaskuläre oder intramyokardiale Zielstelle geeignet ist, und
einen proximalen Katheteransatz (160), der an dem proximalen Ende des Schutzkatheters (150) bereitgestellt ist und zum Leiten des Schutzkatheters (150) durch das Gefäßsystem ausgelegt ist, wobei der proximale Katheteransatz (160) darauf ausgelegt ist, dass die endoluminale Freisetzungskanüle (1) dahindurch und in den Schutzkatheter (150) eingeführt wird.

13. Endoluminale Freisetzungsanordnung (400) nach Anspruch 12, wobei der Katheteransatz (160) ferner ein Verriegelungsmittel (162) umfasst, das darauf ausgelegt ist, reversibel zwischen einem verriegelten Zustand und einem nichtverriegelten Zustand abzuwechseln, wobei das Verriegelungsmittel (162) dazu konfiguriert ist, in einem verriegelten Zustand eine axiale Bewegung zwischen dem Schutzkatheter (150) und der endoluminalen Freisetzungskanüle (1) zu verhindern.

14. Endoluminale Freisetzungsanordnung (400) nach Anspruch 13, wobei das Verriegelungsmittel (162) ein Verriegelungsrad (164) umfasst, das mit inneren Gewinden (165a) bereitgestellt ist, die mit äußeren Gewinden (165b) des Katheteransatzgehäuses zusammenwirken, wobei das Verriegelungsmittel derart ausgelegt ist, dass, wenn das Verriegelungsmittel betätigt wird, eine innere Verriegelungsmanschette (166) zusammengedrückt wird, um ein proximales Ende der endoluminalen Freisetzungskanüle in Eingriff zu nehmen.

## Revendications

1. Canule d'administration endoluminale (1), pour l'administration d'une substance à un site cible extravasculaire ou intramyocardique via le système vasculaire d'un corps humain ou animal,
ladite canule (1) ayant une extrémité proximale (2) configurée pour rester à l'extérieur du corps et une extrémité distale (3) configurée pour être insérée dans le corps via le système vasculaire pour accéder au site cible extravasculaire ou intramyocardique, ladite canule (1) ayant une longueur longitudinale totale (L₁) de ladite extrémité proximale (2) à ladite extrémité distale (3), et ladite canule (1) comprenant
un moyeu de canule (8) prévu au niveau de l'extrémité proximale (2) de la canule (1),
une partie proximale allongée (4) ayant une longueur longitudinale (L₂) et un diamètre externe (D₂), ledit diamètre externe (D₁) étant constant sur sensiblement toute la longueur de la partie proximale (4) telle que mesurée lorsque la canule est sensiblement droite, et
une partie de pointe (5) agencée distalement par rapport à la partie proximale (4) et s'étendant de la partie proximale (4) à une pointe distale (6) de la canule (1), et
une lumière continue (7) s'étendant de l'extrémité proximale (2) de la canule à travers la partie proximale (4) et la partie de pointe (5) à la pointe distale (6) et ladite lumière (7) ayant un diamètre interne (D₂) sur toute la longueur de la canule (1),
ladite partie de pointe (5) ayant une ouverture primaire (9) au niveau de la pointe distale (6) pour assurer une communication entre la lumière (7) et l'extérieur de la canule (1),
ladite partie de pointe (5) étant effilée vers la pointe distale (6) sur toute la partie de pointe (5) de l'extrémité distale de la partie proximale allongée (4) à la pointe distale (6),
ladite partie de pointe effilée (5) étant prévue au niveau de l'extrémité proximale de la partie de pointe (5) avec un diamètre externe (D₃) étant sensiblement le même que le diamètre externe (D₁) de la partie proximale allongée (4), et un diamètre externe (D₄) au niveau de la pointe distale (6) étant plus petit que le diamètre externe (D₃) au niveau de l'extrémité proximale de la partie de pointe effilée (5),
dans laquelle ladite partie de pointe effilée (5) est pourvue d'une section de pointe pointue distale (100) pour pénétrer le tissu,
**caractérisée en ce que**
ladite partie de pointe effilée (5) a une longueur longitudinale (L₃) d'au moins 100 mm, de préférence comprise entre 100 mm et 300 mm, plus préférablement comprise entre 200 mm et 280 mm,
dans laquelle ladite section de pointe pointue (100) comprend au moins une facette primaire (F₁) et deux facettes secondaires (F₂, F₃),
dans laquelle lesdites deux facettes secondaires (F₂, F₃) sont agencées de manière proximale par rapport à ladite facette primaire (F₂), et
dans laquelle ladite section de pointe pointue distale (100) a également une lumière (7) étant une continuation de la lumière continue (7).

2. Canule d'administration endoluminale (1) selon la revendication 1, dans laquelle ladite longueur longitudinale totale (L₁) de ladite extrémité proximale (2) à ladite extrémité distale (3) est comprise dans la plage d'environ 300 mm à 2 500 mm.

3. Canule d'administration endoluminale (1) selon une quelconque revendication précédente, dans laquelle ledit diamètre externe D₄ au niveau de la pointe distale (6) est compris dans la plage de 0,10 mm à 0,25 mm, et de préférence entre 0,15 mm et 0,22 mm.

4. Canule d'administration endoluminale (1) selon une quelconque revendication précédente, dans laquelle ladite partie de pointe effilée (5) et/ou partie proximale (4) est pourvue d'une ou de plusieurs bandes de marquage radio-opaques (11) à des distances prédéfinies de la pointe distale (6).

5. Canule d'administration endoluminale (1) selon une quelconque revendication précédente, dans laquelle ladite partie de pointe effilée (5) est pourvue d'un ou de plusieurs éléments de limite de profondeur saillants (12).

6. Canule d'administration endoluminale (1) selon une quelconque revendication précédente, dans laquelle ladite partie de pointe effilée (5) est en outre pourvue d'une ou de plusieurs ouvertures latérales (9') le long d'au moins une partie de la partie de pointe effilée (5).

7. Canule d'administration endoluminale (1) selon une quelconque revendication précédente, dans laquelle le moyeu de canule (8) est pourvu d'un canal longitudinal interne (10) et d'un connecteur femelle (13) au niveau de son extrémité proximale, ledit canal longitudinal interne (10) étant configuré pour fournir une communication entre la lumière continue (7) de la canule et le connecteur femelle (10), dans laquelle le canal longitudinal interne (10) et le connecteur femelle (13) ont ensemble un volume interne total qui est inférieur à 0,45 ml lorsqu'un connecteur mâle correspondant est fixé au connecteur femelle (13).

8. Canule d'administration endoluminale (1) selon une quelconque revendication précédente, dans laquelle ladite section de pointe distale (100) a
un premier plan (P₁) étant agencé le long d'un axe longitudinal central (A), et un deuxième plan (P₂) étant agencé le long de l'axe longitudinal (A), lesdits premier et deuxième plans (P₁, P₂) étant perpendiculaires l'un à l'autre, et
un troisième plan (P₃) étant positionné selon un angle thêta (θ), par rapport au premier plan (P₁), ledit troisième plan (P₃) étant agencé symétriquement par rapport au deuxième plan (P₂), et
un quatrième plan (P₄) et un cinquième plan (P₅) étant agencés selon un ensemble de deux angles symétriques, lesdits angles étant symétriques par rapport à chacun desdits premier et deuxième plans (P₁, P₂),
lesdits angles symétriques étant des angles combinés comprenant un premier angle phi (ϕ) tel que mesuré à partir du deuxième plan (P₂) dans le premier plan (P₁), et un second angle oméga (ω) étant un angle de rotation autour de l'axe longitudinal (A),
dans laquelle
ladite facette primaire (F₁) de ladite section de pointe distale (100) est prévue dans ledit troisième plan (P₃), et
lesdites deux facettes secondaires (F₂, F₃) sont prévues respectivement dans lesdits quatrième et cinquième plans (P₄, P5) .

9. Canule d'administration endoluminale (1) selon la revendication 8, dans laquelle lesdites deux facettes secondaires (F₂, F₃) forment une pointe distale (6) avec une surface d'enveloppe externe de la section de pointe (100).

10. Canule d'administration endoluminale (1) selon l'une quelconque des revendications 8 ou 9, dans laquelle l'angle phi (ϕ) est plus grand que ledit angle thêta (θ).

11. Canule d'administration endoluminale (1) selon l'une quelconque des revendications 8 à 10, dans laquelle ladite facette primaire (F₁) et lesdites deux facettes secondaires (F₂, F₃) sont prévues selon un angle de facette primaire thêta (θ) qui est compris entre 10,0 et 20,0 degrés, et les facettes secondaires étant prévues selon des angles +/- phi (ϕ) compris entre 15,0 et 20,0 degrés, et des angles +/- oméga (ω) compris entre 25,0 et 90,0 degrés.

12. Ensemble d'administration endoluminale (400), pour l'administration d'une substance à un site cible extravasculaire ou intramyocardique via le système vasculaire d'un corps humain ou animal, comprenant
la canule d'administration endoluminale (1) selon l'une quelconque des revendications 1 à 11,
un cathéter protecteur (150) conçu pour être inséré dans le système vasculaire d'un corps humain ou animal,
dans lequel une extrémité distale dudit ensemble est configurée pour être guidée vers une position dans le système vasculaire appropriée pour accéder au site cible extravasculaire ou intramyocardique prévu, et
un moyeu de cathéter proximal (160) prévu au niveau de l'extrémité proximale du cathéter protecteur (150) et conçu pour guider le cathéter protecteur (150) à travers le système vasculaire, ledit moyeu de cathéter proximal (160) étant conçu pour que la canule d'administration endoluminale (1) soit insérée à travers celui-ci et dans ledit cathéter protecteur (150) .

13. Ensemble d'administration endoluminale (400) selon la revendication 12, dans lequel le moyeu de cathéter (160) comprend en outre un moyen de verrouillage (162) conçu pour alterner de manière réversible entre un état verrouillé et un état déverrouillé, ledit moyen de verrouillage (162) étant configuré, dans un état verrouillé, pour empêcher un mouvement axial entre le cathéter protecteur (150) et la canule d'administration endoluminale (1).

14. Ensemble d'administration endoluminale (400) selon la revendication 13, dans lequel le moyen de verrouillage (162) comprend une roue de verrouillage (164) pourvue de filetages internes (165a) collaborant avec des filetages externes (165b) du boîtier de moyeu de cathéter, ledit moyen de verrouillage étant conçu de sorte que lorsque ledit moyen de verrouillage est actionné, un joint de verrouillage interne (166) est comprimé pour saisir une extrémité proximale de ladite canule d'administration endoluminale.
